# EUROPÄISCHE PATENTSCHRIFT

(11) **EP 0 637 313 B1**
(45) Veröffentlichungstag und Bekanntmachung des Hinweises auf die Patenterteilung: **05.03.1997**
(21) Anmeldenummer: 93911764.4
(22) Anmeldetag: 16.04.1993
(51) Int. Cl.: C07H 15/04

(54) **VERFAHREN ZUM DESTILLATIVEN ABTRENNEN VON ALKOHOLEN**
PROCESS FOR THE DISTILLATORY SEPARATION OF ALCOHOLS
PROCEDE DE SEPARATION D'ALCOOLS PAR DISTILLATION

(30) Priorität: 24.04.1992 DE 4213456
(43) Veröffentlichungstag der Anmeldung: 08.02.1995
(73) Patentinhaber: Henkel Kommanditgesellschaft auf Aktien, 40191 Düsseldorf (DE)
(72) Erfinder: JOHANNISBAUER, Wilhelm, D-4006 Erkrath (DE); NITSCHE, Michael, D-5650 Solingen 1 (DE); JEROMIN, Lutz, D-4010 Hilden (DE)
(86) Internationale Anmeldenummer: EP9300929
(87) Internationale Veröffentlichungsnummer: WO9322323

(56) Entgegenhaltungen:
- EP-A- 0 377 831
- EP-A- 0 418 458
- EP-A- 0 421 187

## Beschreibung

Die Erfindung betrifft ein Verfahren zum ein- oder mehrstufigen destillativen Abtrennen von aliphatischen Alkoholen mit bis zu 30, insbesondere mit 6 bis 18 Kohlenstoffatomen aus einem Gemisch von Alkylglycosiden und bei der Herstellung dieser Alkylglycoside nicht umgesetzten Alkoholen.

Der Begriff Alkylglycoside wird im folgenden für die Reaktionsprodukte aus beliebigen Zuckerarten und aliphatischen Alkoholen verstanden. Als Zucker kommen Monosaccharide, wie Pentosen und Hexosen, Disaccharide, wie Saccharose und Maltose, und Polysaccharide wie Stärke in Betracht. Vorzugsweise werden wegen der besseren Reaktionsfähigkeit die Aldosen verwendet. Unter den Aldosen ist wegen ihrer leichten Zugänglichkeit und Verfügbarkeit in technischen Mengen die Glucose bevorzugt. Der Begriff Alkyl in Alkylglykosid umfaßt im weitesten Sinne den Rest eines aliphatischen Alkohols beliebiger Kettenlänge, vorzugsweise eines primären aliphatischen Alkohols und insbesondere eines aus natürlichen Fetten erhältlichen Fettalkohols, so daß der Begriff gesättigte und ungesättigte Reste und deren Gemische einschließlich solcher mit verschiedenen Kettenlängen im Gemisch umfaßt.

Die Kohlenhydrate werden mit den Alkoholen in Gegenwart eines geeigneten sauren Katalysators umgesetzt. Dabei erhält man Gemische aus Alkylmonoglycosiden und Alkylpoly- oder Alkyloligoglycosiden. Die Begriffe Alkyloligoglykosid, Alkylpolyglykosid, Alkyloligosaccharid und Alkylpolysaccharid beziehen sich auf solche alkylierten Glykosen, in denen ein Alkylrest in Form des Acetals an mehr als einen Glykoserest, also an einen Poly- oder Oligosaccharidrest gebunden ist. Diese Begriffe werden als untereinander gleichbedeutend angesehen. Dementsprechend ist ein Alkylmonoglykosid das Acetal eines Monosaccharids. Da man bei der säurekatalysierten Umsetzung von Zuckern und Fettalkoholen im allgemeinen Gemische erhält, werden im folgenden unter dem Begriff Alkylglykosid sowohl Alkylmonoglykoside als auch Alkylpoly(oligo)glykoside und insbesondere Mischungen daraus - einschließlich eventueller Nebenkomponenten - verstanden, sofern es nicht ausrücklich auf die strukturellen Unterschiede ankommt.

Als Herstellungsverfahren für Alkylglykoside ist zum einen die direkte Umsetzung der Glykose, meist in Form von Glucose, mit einem Überschuß des Fettalkohols und einer Säure als Katalysator (Direktsynthese), und zum andern die Mitverwendung eines niederen Alkohols oder Glykols als Lösungsmittel und Reaktionspartner (Umacetalisierung) bekannt. Die Reaktion wird in der Regel mit einem großen Überschuß an Alkohol durchgeführt, so daß man als Reaktionsprodukt ein Gemisch aus den Alkylglycosiden und Alkoholen erhält. Zur Verbesserung der anwendungstechnischen Eigenschaften der Alkylglycoside werden die Alkohole üblicherweise aus dem Gemisch bis auf wenige Gew.-% oder bis auf unter 1 Gew.-% abgetrennt. Anschließend empfiehlt sich aus wirtschaftlichen Gründen eine Rückführung der Alkohole in den Reaktor.

Ein Problem, das bei der Herstellung der oberflächenaktiven Alkylglykoside auf Basis der üblichen Fettalkohole mit 12 bis 18 Kohlenstoffatomen auftritt, liegt in der Schwierigkeit der destillativen Abtrennung des nichtumgesetzten Anteils dieser Fettalkohole aus dem Reaktionsprodukt. Dazu wird in der europäischen Patentanmeldung 32 252 vorgeschlagen, die destillative Abtrennung dieser nichtumgesetzten Fettalkohole in Gegenwart von Schleppmitteln, nämlich solchen Glykolen durchzuführen, deren Siedepunkte die der abzutrennenden Alkohole um höchstens 10 °C über- und um höchstens 30 °C unterschreiten. Auf diese Weise kann die Destillation bei nicht mehr als 140 °C und einem Vakuum von etwa 8 mbar, d. h. in einer für das Produkt schonenden Weise durchgeführt werden. Die Zugabe von Schleppmitteln weist aber den Nachteil auf, daß das Produkt mit zusätzlichen Stoffen in Kontakt kommt, die einerseits die Produktqualität mindern können und die andererseits einen vermehrten apparativen Aufwand für die Abtrennung und Rückführung des Schleppmittels bedingen.

In einem anderen bekannten Verfahren zur Herstellung von Alkylglykosiden wird in einem Zwischenschritt mit einem niedrigen Alkohol umacetalisiert. Nach der europäischen Patentanmeldung 92 875 wird das Umacetalisierungsverfahren mit Butanol zur Herstellung von langkettigen Alkylglucosiden so gesteuert, daß noch eine Restmenge an Butylglucosiden von weniger als 10 Gew.-% im Verfahrensprodukt enthalten ist. Auf diese Weise wird die Bildung der langkettigen Alkyloligoglucoside mit höherem Oligomerisierungsgrad, d. h. mit 6 und mehr Glucoseeinheiten im Molekül verringert. Die so erhaltenen Produkte bestehen im wesentlichen aus Alkylmonoglucosid und Alkyloligoglucosiden, wobei der Mengenanteil der Alkylmonoglucoside maximal 60 Gew.-% und der durchschnittliche Oligomerisierungsgrad 1,5 bis 3 beträgt. Der Anteil an kurzkettigen Alkylglucosiden, insbesondere Butylglucosiden, liegt unterhalb 10 %, der Anteil an nichtumgesetztem Fettalkohol soll unter 2 % liegen. Zur destillativen Entfernung des Fettalkohols wird die Benutzung eines Dünnschichtverdampfers (wiped film evaporator) empfohlen.

Für die schonende Auftrennung von temperaturempfindlichen Substanzgemischen gilt generell, daß sich zur schonenden Verdampfung bei reduziertem Druck Fallfimverdampfer und insbesondere Dünnschichtverdampfer besonders gut eignen, weil sich in diesen Geräten extrem kurze Verweilzeiten bei den erforderlichen höheren Temperaturen erreichen lassen. Als Dünnschichtverdampfer oder "wiped film evaporator" bezeichnet man solche Verdampfer, in denen ein hochviskoses schwer siedendes Gemisch auf eine beheizte Wand aufgegeben und dort durch rotierende Wischelemente mechanisch verteilt wird. Dabei werden dünne geschlossene Flüssigkeitsschichten bzw. Flüssigkeitsfilme erzeugt, und die Filmoberflächen werden ständig erneuert, so daß lokale Überhitzungen vermieden werden. Die entstehenden Dämpfe strömen entgegen dem Fluß des Produktfilms und verlassen den Verdampfer in den außen angeordneten Kondensator. Im Dünnschichtverdampfer wird im allgemeinen bei Drucken von nur einigen mbar gearbeitet, und die Verweildauer für das Produkt beträgt nur wenige Sekunden.

Die europäische Patentanmeldung 92 876 beschreibt ebenfalls die Herstellung von langkettigen Alkylglucosiden mit einem Oligomerisierungsgrad von 1,5 bis 20 nach dem Umacetalisierungsverfahren mit Butanol, wobei der die Umacetalisierung bewirkende Katalysator (Paratoluolsulfonsäure) durch Neutralisation dann inaktiviert wird, wenn wenigstens 90 % des Butylglucosids reagiert haben, so daß noch höchstens 10 % Butylglucosid im Reaktionsprodukt verbleiben. Zur schonenden Entfernung des Fettalkoholüberschusses wird hier ebenfalls die Verwendung eines Dünnschichtverdampfers empfohlen. Wichtig dabei ist die Anwesenheit von geringen Mengen von Butylglycosid, um die Viskosität des Gemisches herabzusetzen und damit niedrigere Temperaturen zu erlauben. Die Reaktionsprodukte sollen ebenfalls weniger als 2 % freien Fettalkohol enthalten.

Zur Beurteilung der destillativen Abtrennverfahren ist außerdem wichtig, daß bei der thermischen Beanspruchung insbesondere die Verweildauer bei der erhöhten Temperatur und weniger deren Höhe selbst maßgeblich ist.

Die thermisch schonende Abtrennung der Fettalkohole aus dem Reaktionsgemisch ist nach diesem Stand der Technik nur bei Anwesenheit von Alkylglucosiden mit kurzer Kettenlänge des Alkylrestes (C₁ bis C₅), also nur bei aus der Umacetalisierungsroute erhaltenen Reaktionsgemischen möglich. Der Einsatz des Dünnschichtverdampfers zur Alkoholabtrennung in der oben genannten Direktsynthese führt daher zu Problemen mit der Fließfähigkeit des Reaktionsgemisches und ist nicht ohne weiteres möglich.

Ein weiteres Problem liegt in der durch die beweglichen Einbauten des Dünnschichtverdampfers herabgesetzten Betriebssicherheit und Standzeit.

Auch in anderen bekannten Verfahren zur Abtennung des Fettalkohols aus dem Reaktionsgemisch wird, zumindest in der Schlußphase der Destillation, ein Dünnschichtverdampfer eingesetzt, bei dem die Flüssigkeit durch Wischer mechanisch auf der Heizfläche verteilt wird (EP 0 301 298 A1, W0 90/039 77, W0 91/048 80).

Die Aufgabe der Erfindung bestand in der Verbesserung der Verfahren zur Abtrennung der Fettalkohole. Das Verfahren soll auch auf aus der Direktsynthese erhaltene Reaktionsgemische anwendbar sein, im technischen Maßstab mit Apparaten ohne mechanisch bewegte Teile arbeiten und bei vertretbaren Gesamtkosten den Alkoholgehalt im Produkt auf beliebig wählbare Werte zwischen 0,1 und 5 Gew.-% absenken, ohne die Qualität des Endproduktes zu beeinträchtigen.

Diese Aufgabe wird erfindungsgemäß dadurch gelöst, daß die Alkohole mit einem Fallfilmverdampfer zum Abreichern des Reaktionsgemisches auf Restalkoholwerte von 5 Gew.-% und weniger abtrennt, wobei man eine Linienbelastung des Fallfilmverdampfers von mindestens 3,0 m³ Feed pro Stunde und pro m Umfangslänge der Verdampferrohre einstellt. Die Linienbelastung, die auch spezifischer Drucksatz genannt wird, ergibt sich dabei aus dem Volumen der pro Zeiteinheit auf die Verdampferrohre aufgegebenen Flüssigkeit, dividiert durch die innere Umfangslänge, die aus dem Produkt des Umfangs eines Einzelrohres und der Anzahl der Verdampferrohre erhältlich ist.

Beim Fallstrom- oder Fallfilmverdampfer wird die Flüssigkeit von oben mit besonderen Vorrichtungen auf die Verdampferrohre verteilt und strömt mit dem entstehenden Dampf nach unten in einen Abscheider für Destillat und Konzentrat. Die Flüssigkeit läuft in einem möglichst geschlossenem Film üblicherweise an den Innenwänden einer Vielzahl von aufrecht stehenden Rohren hinunter, die von außen beheizt werden. Für die Ausbildung eines in allen Rohren gleichmäßigen und geschlossenen Films ist eine gleichmäßige Verteilung des Einsatzproduktes auf die Rohre erforderlich. Der Verzicht auf mechanisch bewegte Teile erhöht die Betriebssicherheit des Fallfilmverdampfers.

Die geforderten anwendungstechnischen Eigenschaften der Alkylglycoside verlangen einen Restalkoholgehalt von weniger als etwa 5 %, so daß zumindest in der Schlußphase der Destillation ein hochviskoses Produkt verarbeitet werden muß. Im allgemeinen wird zwar der Einsatz von Dünnschichtverdampfern für derartige hochviskose Produkt, die nur für kurze Verweilzeiten einer erhöhten Temperatur ausgesetzt werden dürfen, für notwendig gehalten. Dennoch gelingt die Abtrennung der Alkohole überraschenderweise auch in einem Fallfilmverdampfer, der bekannterweise eher für niedriger viskose Produkte ausgelegt ist, die außerdem höhere Verweilzeiten zulassen.

Unter üblichen Verfahrensbedingungen wäre jedoch ein Ersatz des Dünnschichtverdampfers durch einen Fallfilmverdampfer nicht möglich, ohne die Qualität des Endproduktes entscheidend zu verschlechtern. Die hohe Viskosität des Einsatzgemisches gegen Ende der Destillation erschwert nämlich die Ausbildung eines gleichmäßigen Flüssigkeitsfilms über die gesamte Wärmeaustauschfläche der Verdampferrohre, insbesondere im unteren Bereich der Rohre. Nicht benetzte Stellen an der Rohrwandung und als deren Folge lokale Überhitzungen und thermische Schädigungen des Produkts würden auftreten, die sich in farbigen Verunreinigungen und Nebenprodukten äußern. Als weitere Folge wären starke Anbackungen, die zu einer Querschnittsverringerung und schließlich zu einem Verstopfen der Rohre führen können, zu nennen.

Erfindungsgemäß werden diese Schwierigkeiten durch eine bestimmte Strömungsführung im Fallfilmverdampfer erreicht, die eine vollständige Benetzung der Wärmeaustauschflächen mit einem gleichmäßigen Flüssigkeitsfilm auch noch im unteren Bereich der Verdampferrohre sicherstellt, so daß lokale Überhitzungen durch Risse im Flüssigkeitsfilm ausgeschlossen sind. Diese Forderung läßt sich trotz der hohen Viskosität durch den genannten hohen Mindestwert für die Linienbelastung erfüllen.

Üblich für große Eindampfungsverhältnisse, d. h. für einen hohen abzutrennenden Destillatanteil, sind jedoch Linienbelastungen von weniger als 1 m³/h m, damit die an der Heizfläche übertragbare Wärmemenge zum Abtrennen des gesamten Leichtsiederanteils ausreicht.

Der Erfindung liegt folgende Erkenntnis zugrunde. Bei der Verarbeitung temperaturempfindlicher Gemische im Fallfilmverdampfer sind eigentlich ein sehr dünner Film und eine niedrige Verweilzeit erwünscht, um die thermische Belastung gering zu halten. Bei der Abtrennung der Alkohole von den Alkylglycosiden ändern sich jedoch die Viskositäten über die Rohrlänge und damit auch die Strömungsverhältnisse sehr stark, so daß es relativ schwierig ist, eine vollständige Benetzung der Wärmeaustauschflächen zu gewährleisten. Die Erfindung baut nun auf einer Abwägung der die thermische Belastung verursachenden Faktoren auf. Die lokalen Überhitzungen infolge von Filmrissen sind erheblich stärker für die thermische Beanspruchung verantwortlich als eine größere Schichtdicke oder eine höhere Verweilzeit, die erfindungsgemäß in Kauf genommen werden. Erfindungswesentlich ist daher die unter allen Umständen sichergestellte Ausbildung eines geschlossenen Flüssigkeitsfilms ohne Risse oder nichtbenetzte Stellen durch das Einhalten der genannten Mindestlinienbelastung.

Das erfindungsgemäße Verfahren läßt sich besonders wirtschaftlich betreiben, wenn man es in mehreren, insbesondere zwei Stufen durchführt und in jeder Stufe als Verdampferapparat einen Fallfilmverdampfer einsetzt. Das Reaktionsgemisch wird in den Vorstufen vorzugsweise auf 5 bis 50 Gew.-%, insbesondere 10 bis 30 Gew.-% Alkohol abgereichert. In der letzten Stufe werden dann Alkoholgehalte von weniger als 10 Gew.-% erreicht.

Das Verfahren läßt sich aber auch einstufig betreiben. Je nach Bauweise des eingesetzten Fallfilmverdampfers der letzten Destillationsstufe kann das erfindungsgemäße Verfahren in hauptsächlich zwei Varianten betrieben werden, von denen jede für unterschiedliche Zielsetzungen besondere Vorteile zeigt.

Das einstufige Verfahren kann mit einfachem Durchlauf des Reaktionsgemisches durch die Verdampferrohre betrieben werden. Der hohe, in nur einer Stufe abzutrennende Destillatanteil erfordert dabei relativ lange Verdampferrohre, um ein Konzentrat mit dem gewünschten maximalen Restalkoholgehalt zu erhalten. Die große Rohrlänge und insbesondere die starke Änderung der Zusammensetzung und der Viskosität der Flüssigkeit und damit der Strömungsverhältnisse entlang des Rohres erfordern einen relativ dicken Flüssigkeitsfilm und eine ungewöhnlich hohe Linienbelastung von mindestens 3,0 m³/h m. Vorteilhaft ist hier die aufgrund des einmaligen Durchlaufs durch das Rohr nur geringe Verweilzeit und damit geringe thermische Belastung.

Eine andere Ausführung des einstufigen Verfahrens bedient sich des äußeren Zwangsumlauf. Das aufzuarbeitende Gemisch wird mittels einer außerhalb des Fallfilmverdampfers angeordneten Umwälzpumpe im Kreis geführt. Ein im Vergleich zur Umlaufrate geringe Menge an rohem Produkt wird in den Kreislauf eingespeist und ein entsprechender Anteil an konzentriertem Produkt ausgeschleust. Der mehrfache Umlauf der Flüssigkeit erlaubt kürzere Rohrlängen als beim einfachen Durchlauf und damit eine kompaktere Bauweise der Anlage oder bei gleicher Anlagengröße einen höheren Durchsatz. Die Zusammensetzung des Flüssigkeitsgemisches ändert sich weniger über die Rohrlänge, und die gleichmäßigeren Strömungsverhältnisse lassen einen dünneren Film und eine niedrigere Linienbelastung von mindestens 1,0 m³/h m bei einem Verhältnis von eingespeister zu umlaufender Flüssigkeitsrate von 1:100 bis 1:5 zu. Die Linienbelastung berechnet sich in diesem Fall aus der gesamten, auf den Verdampfer aufgegebenen Flüssigkeitsmenge, also der umgewälzten Menge. Trotz der geringeren Filmdicke bleibt die vollständige Benetzung der Rohrwand mit der aufgegebenen Flüssigkeit über die gesamte Rohrlänge gewährleistet.

Je nach Anforderung an den Durchsatz und je nach Temperaturempfindlichkeit des Produktes ist es also vorteilhaft, die einstufige Destillation entweder mit oder ohne äußeren Zwangsumlauf zu betreiben.

Im folgenden werden besonders bevorzugte Drücke und Temperaturen zum Betreiben des erfindungsgemäßen Verfahrens genannt.

Bei mehrstufigen Prozessen wird für die der letzten Verdampferstufe vorangehenden Stufen bevorzugt eine Sumpfablauftemperatur von 100 bis 220 °C, insbesondere von 140 bis 200 °C sowie ein Betriebsdruck von 0,5 bis 20 mbar, insbesondere 1 bis 10 mbar eingestellt.

In einstufigen Verfahren und in der letzten Verdampferstufe mehrstufiger Verfahren arbeitet man vorteilhaft mit Sumpfablauftemperaturen von 120 bis 250 °C, insbesondere 160 bis 230 °C und einem Betriebsdruck von 0,1 bis 10 mbar, insbesondere von 0,5 bis 5 mbar.

Im Anschluß an die Entfernung des Fettalkohol-Überschusses wird das Reaktions-Endprodukt, das im erkalteten Zustand eine hellbraune wachsartige Masse bildet, vorzugsweise wegen der besseren Handhabbarkeit in eine wäßrige Paste mit ca. 60 % Wirkstoffgehalt übergeführt. Wenn hohe Ansprüche an die Farblosigkeit des Endprodukts gestellt werden, kann gleichzeitig mit der Herstellung der wäßrigen Paste oder im Anschluß daran eine Bleiche mit Wasserstoffperoxid oder einer organischen Persäure, wie z. B. Dodecandipersäure vorgenommen werden.

Nachfolgend werden Ausführungsbeispiele der Erfindung erläutert, ohne daß die Erfindung darauf beschränkt ist.

### Beispiele

### Beispiel 1: Zweistufige Destillation mit Flüssigkeitsumlauf

In einer zweistufigen Destillationsanlage, bestehend aus einem ersten Fallfilmverdampfer mit 2,5 m² Verdampferfläche und einem zweiten, nachgeschalteten Fallfilmverdampfer mit 4,5 m² Verdampferfläche, wurde ein Gemisch aus Alkylglycosiden (APG) und Fettalkoholen getrennt. Die Alkylglucoside waren aus Glucosesirup und einem Gemisch aus Laurin- und Myristinalkohol synthetisiert worden, wobei das Molverhältnis von Glycose zu Alkohol 1 : 4,9 betragen hatte.

Der erste Fallfilmverdampfer wurde mit einer Heizmitteltemperatur von 180 °C, einer Sumpfablauftemperatur von 160 °C und einem Druck von etwa 2 mbar betrieben. Der zweite Fallfilmverdampfer wurde mit einer Heizmitteltemperatur von 210 °C, einer Sumpfablauftemperatur von 200 °C und einem Druck von etwa 2 mbar betrieben.

In der ersten Destillationsstufe wurde der Fettalkoholgehalt auf etwa 10 Gew.-% reduziert. Die Flüssigkeitsaufgabe und die gleichmäßige Benetzung der Rohrwände in der ersten Verdampfungsstufe, in der ein relativ großer Destillatanteil anfiel, wurde mit einem äußeren Zwangsumlauf erleichtert. Im stationären Betriebszustand wurden 4,0 m³/h umgewälzt. Eingespeist wurden 0,3 m³/h. Die Linienbelastung betrug hier 4,8 m³/h m.

In die zweite Destillationsstufe wurde im stationären Betrieb 0,12 m³/h aus der ersten Stufe eingespeist und 5,0 m³/h umgewälzt. Auch hier wurde mit äußerem Zwangsumlauf gearbeitet. Die Linienbelastung betrug 3,0 m³/h m. Der Fettalkoholgehalt wurde auf 0,7 Gew.-% herabgesetzt.

Es wurde eine hellbraune Masse erhalten, die nach dem Überführen in eine wäßrige Paste mit etwa 60 % Gehalt an APG zu einem hellgelben bis fast farblosen Produkt gebleicht werden konnte. Das Produkt war alkalistabil.

### Beispiel 2: Einstufige Destillation mit Flüssigkeitsumlauf

Der Versuch wurde wie in Beispiel 1, aber mit einstufiger Verdampfung in einem Fallfilm-Apparat mit 2,5 m² Verdampferfläche bei einem Einsatzmassenstrom von 0,05 m³/h, einer Heizmitteltemperatur von 210 °C, einer Sumpfablauftemperatur von 200 °C und einem Druck von etwa 2 mbar durchgeführt. Die Anlage wurde mit äußerem Zwangsumlauf betrieben, wobei 4,0 m³/h umgewälzt wurden. Die Linienbelastung betrug 4,8 m³/h m.

Die erhaltene hellbraune Masse hatte einen Fettalkoholgehalt von 0,8 Gew.-%. Die entsprechende wäßrige mit Wasserstoffperoxid gebleichte Paste mit einem APG-Gehalt von 60 % war hellgelb bis fast farblos.

### Beispiel 3: Einstufige Destillation mit Flüssigkeitsumlauf

Im gleichen Apparat wie in Beispiel 2 wurde eine einstufige Destillation mit äußerem Zwangsumlauf bei einem Einsatzmassenstrom von 0,05 m³/h und 4,0 m³/h Umwälzmenge durchgeführt. Eingesetzt wurde ein Alkylpolyglycosid auf Basis von Glucose sowie einem Gemisch aus Capryl- und Caprinalkohol, wobei das Molverhältnis von Glucose und Alkohol in der Reaktion 1 : 2,5 betrug. Es wurde mit einer Heizmitteltemperatur von 180 °C, einer Sumpfablauftemperatur von 170 °C, einem Druck von 2 mbar und einer Linienbelastung von 4,8 m³/h m gearbeitet.

Der Fettalkoholgehalt des Konzentrats betrug 0,5 Gew.-%. Das in eine wäßrige Paste mit etwa 70 % APG-Gehalt überführte und mit Wasserstoffperoxid gebleichte Produkt war hellgelb bis fast farblos.

### Beispiel 4 (Vergleichsbeispiel)

Gleiche Bedingungen wie in Beispiel 2, d.h. 2,5 m² Fläche, 0,05 m³/h Einsatzmassenstrom, 210 °C Heizmitteltemperatur, 2 mbar Druck wurden eingehalten.

Die Verringerung der Umwälzmenge von 4,0 m³/h (entsprechend einer Linienbelastung von 4,8 m³/h m) auf 0,6 m³/h (Linienbelastung einschließlich Einsatzmassenstrom 0,78 m³/h m) führte zu einem dunkelbraunen Produkt mit einem Fettalkoholgehalt von 2,5 bis 4 Gew.-%.

Eine weitere Verringerung der Umwälzmenge auf 0,3 m³/h (Linienbelastung inkl. Einsatzmassenstrom 0,42 m³/h m) führte zu einem dunkelbraunen bis schwarzen Produkt mit einem Fettalkoholgehalt von 4 bis 5 Gew.-%.

## Patentansprüche

1. Verfahren zum ein- oder mehrstufigen destillativen Abtrennen von aliphatischen Alkoholen mit bis zu 30, insbesondere mit 6 bis 18 Kohlenstoffatomen, aus einem Gemisch von Alkylglycosiden und bei der Herstellung dieser Alkylglycoside nicht umgesetzten Alkoholen mit einem Fallfilmverdampfer zum Abreichern des Reaktionsgemisches auf Restalkoholwerte von 5 Gew.-% und weniger, wobei man eine Linienbelastung des Fallfilmverdampfers von mindestens 1,0 m³/h und m Gesamtumfang der Rohre, insbesondere von mindestens 1,8 m³/h m und besonders bevorzugt von mindestens 3,0 m³/h m einstellt.

2. Verfahren nach Anspruch 1,
**dadurch gekennzeichnet,**
daß man es in mehreren, insbesondere zwei Stufen durchführt und in jeder Stufe als Verdampferapparat einen Fallfilmverdampfer einsetzt.

3. Verfahren nach Anspruch 2,
**dadurch gekennzeichnet,**
daß man das Reaktionsgemisch in den Vorstufen auf 5 bis 50 Gew.-%, insbesondere 10 bis 30 Gew.-% Alkohol abreichert.

4. Verfahren nach Anspruch 1,
**dadurch gekennzeichnet,**
daß man das Verfahren mit nur einer Stufe betreibt.

5. Verfahren nach Anspruch 4,
**dadurch gekennzeichnet,**
daß man das Reaktionsgemisch nur einmal durch den Fallfilmverdampfer laufen läßt, wobei man eine Linienbelastung von mindestens 3,0 m³/h m einstellt.

6. Verfahren nach Anspruch 4,
**dadurch gekennzeichnet,**
daß man das Verfahren mit äußerem Zwangsumlauf betreibt und ein Verhältnis von eingespeister zu umlaufender Flüssigkeitsrate von 1:100 bis 1:5 und eine Linienbelastung von mindestens 1,0 m³/h m einstellt.

7. Verfahren nach einem der Ansprüche 1 bis 3,
**dadurch gekennzeichnet,**
daß man in einem mehrstufigen Verfahren eine Sumpfablauftemperatur von 100 bis 220 °C, insbesondere von 140 bis 200 °C sowie einen Betriebsdruck von 0,5 bis 20 mbar, insbesondere 1 bis 10 mbar für die der letzten Stufe vorangehenden Stufen einstellt.

8. Verfahren nach einem der Ansprüche 1 bis 7,
**dadurch gekennzeichnet,**
daß man in einstufigen Verfahren und in der letzten Verdampferstufe mehrstufiger Verfahren mit Sumpfablauftemperaturen von 120 °C bis 250 °C, insbesondere 160 bis 230 °C und einem Betriebsdruck von 0,1 bis 10 mbar, insbesondere von 0,5 bis 5 mbar arbeitet.

## Claims

1. A process for the single-stage or multistage separation of aliphatic alcohols containing up to 30 carbon atoms and, more particularly, 6 to 18 carbon atoms by distillation from a mixture of alkyl glycosides and alcohols remaining unreacted in the production of these alkyl glycosides with a falling film evaporator for stripping the reaction mixture to residual alcohol contents of 5% by weight or lower, a linear load of the falling film evaporator of at least 1.0 m³/h and m total circumference of the tubes, preferably of at least 1.8 m³/h m and, more preferably, of at least 3.0 m³/h m being established.

2. A process as claimed in claim 1, characterized in that it is carried out in several stages, more particularly two stages, and a falling film evaporator is used as the evaporator in each stage.

3. A process as claimed in claim 2, characterized in that the reaction mixture is reduced in the preliminary stages to an alcohol content of 5 to 50% by weight and, more particularly, 10 to 30% by weight.

4. A process as claimed in claim 1, characterized in that the process is carried out in only one stage.

5. A process as claimed in claim 4, characterized in that the reaction mixture is passed only once through the falling film evaporator, a linear load of at least 3.0 m³/h m being established.

6. A process as claimed in claim 4, characterized in that the process is carried out with forced external circulation and a ratio of feed to circulating liquid of 1:100 to 1:5 and a linear load of at least 1.0 m³/h m are established.

7. A process as claimed in any of claims 1 to 3, characterized in that, in a multistage process, a sump temperature of 100 to 220°C and, more particularly, 140 to 200°C and an operating pressure of 0.5 to 20 mbar and, more particularly, 1 to 10 mbar are established for the stages preceding the last stage.

8. A process as claimed in any of claims 1 to 7, characterized in that single-stage processes and the last evaporator stage of multistage processes are carried out at sump temperatures of 120 to 250°C and, more particularly, 160 to 230°C and under operating pressures of 0.1 to 10 mbar and, more particularly, 0.5 to 5 mbar.

## Revendications

1. Procédé de séparation par distillation en une ou plusieurs étapes d'alcools aliphatiques ayant jusqu'à 30, notamment de 6 à 18 atomes de carbone, d'un mélange d'alkylglycosides et des alcools qui n'ont pas réagi lors de la préparation de ces alkylglycosides avec un évaporateur à ruissellement pour diminuer la concentration en alcool résiduel du mélange réactionnel à des valeurs de 5 % en poids et moins, en ajustant une charge en ligne de l'évaporateur à ruissellement d'au moins 1,0 m³/h et m de périmètre de tubes, notamment d'au moins 1,8 m³/h m et surtout d'au moins 3,0 m³/h.

2. Procédé selon la revendication 1,
caractérisé en ce qu'
on le réalise en plusieurs étapes notamment deux et qu'à chaque étape on utilise un évaporateur à ruissellement comme évaporateur.

3. Procédé selon la revendication 2,
caractérisé en ce qu'
on diminue la concentration en alcool résiduel du mélange réactionnel dans les premières étapes entre 5 et 50 % en poids, notamment entre 10 à 30 % en poids d'alcool.

4. Procédé selon la revendication 1,
caractérisé en ce qu'
on réalise le procédé en une étape seulement.

5. Procédé selon la revendication 4,
caractérisé en ce qu'
on fait passer le mélange réactionnel seulement une fois dans l'évaporateur à ruissellement, en ajustant une charge en ligne d'au moins 3,0 m³/h m.

6. Procédé selon la revendication 4,
caractérisé en ce qu'
on fait fonctionner le procédé avec une circulation forcée extérieure et un rapport de débit injecté au débit circulant de 1:100 à 1:5 et qu'on ajuste une charge en ligne d'au moins 1,0 m³/h m.

7. Procédé selon l'une des revendications 1 à 3,
caractérisé en ce qu'
on ajuste, dans un procédé à plusieurs étapes une température de soutirage de pied de 100 à 220°C, notamment de 140 à 200°C, ainsi qu'une pression de service de 0,5 à 20 mbar, notamment 1 à 10 mbars dans les étapes qui précèdent la dernière étape.

8. Procédé selon l'une des revendications 1 à 7,
caractérisé en ce que
dans un procédé à une étape et dans la dernière étape d'un procédé à plusieurs étapes on travaille avec des températures de soutirage de 120°C à 250°C, notamment 160 à 230°C sous une pression de service de 0,1 à 10 mbars, notamment de 0,5 à 5 mbars.
